(19) Europäisches Patentamt
European Patent Office
Office européen des brevets

(11) **EP 4 736 941 A1**

(12) **EUROPEAN PATENT APPLICATION**

(43) Date of publication:
06.05.2026 Bulletin 2026/19

(21) Application number: 25212653.7

(22) Date of filing: 31.10.2025

(51) International Patent Classification (IPC):
**A61N 5/10** (2006.01)    **G21K 1/04** (2006.01)

(52) Cooperative Patent Classification (CPC):
**A61N 5/1045; A61N 5/1036; G21K 1/04; G21K 1/046**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: 31.10.2024  CN 202411548308

(71) Applicant: Shanghai United Imaging Healthcare Co., Ltd.
Shanghai 201807 (CN)

(72) Inventors:
• ZHANG, Jian
Shanghai, 201807 (CN)
• Song, Bin
Shanghai, 201807 (CN)
• QU, Yixiang
Shanghai, 201807 (CN)

(74) Representative: Wang, Bo
Panovision IP
Ebersberger Straße 3
85570 Markt Schwaben (DE)

(54) **MULTI-LEAF COLLIMATORS, RADIATION TREATMENT DEVICES, AND CONTROL METHODS THEREOF**

(57) An MLC is provided, the MLC includes: a plurality of leaves; first driving mechanisms connected to the leaves and configured to drive the leaves to move to form a radiation field; and at least one leaf head mechanism mounted on at least one corresponding leaf of the leaves and configured to adjust the radiation field.

**FIG. 1A**

EP 4 736 941 A1

FIG. 1B

**Description**

**TECHNICAL FIELD**

The present disclosure relates to the field of radiation treatment technology, and in particular, relates to a multi-leaf collimator, a radiation treatment device, and a control method thereof.

**BACKGROUND**

Radiation treatment is one of the most important methods for treating tumors, and precise radiation treatment helps improve treatment effect while reducing harm to patients during treatment. A multi-leaf collimator (MLC) is a core device enabling precise radiation treatment, implementing a conformal and intensity-modulated therapy, and enhancing therapeutic outcomes. The MLC with a high resolution helps doctors to target a treatment region more precisely and minimize damage to surrounding healthy tissues in the radiation treatment, which improves the treatment effectiveness and reduces side effects. However, due to the constraints of the MLC's transmission mechanism, the thickness of its leaves cannot be made too thin, which often results in relatively low resolution for the MLC.

Therefore, it is desirable to provide an MLC with a high resolution, a radiation treatment device including such MLC, and a control method thereof.

**SUMMARY**

According to an aspect of the present disclosure, a multi-leaf collimator is provided. The multi-leaf collimator comprises a plurality of leaves; a first driving mechanism connected to the leaves and configured to drive the leaves to move to form a radiation field; and at least one leaf head mechanism mounted on at least one corresponding leaf of the leaves and configured to adjust the radiation field.

In some embodiments, each of the leaves has at least one corresponding leaf head mechanism.

In some embodiments, each leaf head mechanism includes leaf heads, each of which has a thickness less than a thickness of the corresponding leaf; and a second driving mechanism configured to drive the leaf heads to move relative to the corresponding leaf.

In some embodiments, the corresponding leaf includes a first end close to the radiation field center, a second end away from the radiation field center, a third end close to a radiation source, and a fourth end away from the radiation source, wherein the leaf head mechanism is mounted on the first end or the fourth end.

In some embodiments, the leaf head mechanism is mounted on the first end, and the first end includes a first recess member. The first recess member includes first motion guide grooves disposed inside. Each leaf head includes a first protrusion member matching the first recess member. The first motion guide grooves are configured to mount the first protrusion members of the leaf heads and limit a motion direction of the leaf heads.

In some embodiments, the first end further includes an extension member closer to the radiation field center relative to the first recess member. Each leaf head further includes an abutting member closer to the radiation field center relative to its first protrusion member, and the second driving mechanism is mounted on the extension member and connected to the abutting members of the leaf heads to drive the leaf heads to move relative to the corresponding leaf.

In some embodiments, the leaf head mechanism is mounted on the fourth end, and the fourth end includes a suspension member suspended on the fourth end of the corresponding leaf. The suspension member includes a second recess member, and the second recess member includes second motion guide grooves disposed inside. Each leaf head includes a second protrusion member matching the second recess member, and the second motion guide grooves are configured to mount the second protrusion members of the leaf heads and limit a motion direction of the leaf heads.

In some embodiments, the second driving mechanism is mounted on a recess bottom surface of the second recess member and connected to the second protrusion members of the leaf heads to drive the leaf heads to move relative to the corresponding leaf.

In some embodiments, the leaves include radiation shielding material, and the radiation field is formed by the leaves.

In some embodiments, the second driving mechanism includes a base, a control film mounted on the base, and a control power supply. The control film is connected to the leaf heads. The control power supply is configured to apply a voltage to the control film to cause the control film to produce a target deformation, and the target deformation of the control film drives the leaf heads to move.

In some embodiments, the control film comprises a plurality of control sub-films arranged in parallel, and the control power supply is configured to individually apply the voltage to each control sub-film.

In some embodiments, the control film includes at least one of an ion-exchange polymer metal composite (IPMC) or a memory alloy material.

In some embodiments, the multi-leaf collimator further includes a processing device and sensors. Each of the sensors is mounted on one of the leaf heads, and the processing device is configured to determine an initial magnitude of the voltage applied by the control power source based on a response model of the control film and second target displacements of the leaf heads, wherein the response model reflects a relationship between the voltage and a mechanical deformation of the control film; obtain actual displacements of the leaf heads collected by the sensors after motions of the leaf heads; and determine an updated magnitude of the voltage based on the second target displacements and the actual displacements of the leaf heads.

In some embodiments, the leaf heads are configured to move collectively with (move along with) the corresponding leaf driven by the first driving mechanism, and/or the leaf heads are configured to move independently driven by the second driving mechanism while the corresponding leaf is stationary, and the second driving mechanism is different from the first driving mechanism.

In some embodiments, the second driving mechanism is configured to individually drive each leaf head to move.

In some embodiments, the multi-leaf collimator further includes second leaves closer to a radiation source than the leaves; the second leaves include radiation shielding material, and the radiation field being formed by the second leaves.

According to an aspect of the present disclosure, a radiation therapy device is provided. The radiation therapy device includes the multi-leaf collimator.

According to an aspect of the present disclosure, a double-layer multi-leaf collimator is provided. The double-layer multi-leaf collimator includes an upper multi-leaf collimator and a lower multi-leaf collimator, wherein the upper multi-leaf collimator is closer to a radiation source than the lower multi-leaf collimator, and each of the upper multi-leaf collimator and the lower multi-leaf collimator is the multi-leaf collimator.

In some embodiments, the double-layer multi-leaf collimator further comprises a processing device configured to determine first target displacements of the leaves of the upper multi-leaf collimator based on contour information of a target radiation field corresponding to a target object; determine third target displacements of the leaves of the lower multi-leaf collimator based on the first target displacements of the leaves of the upper multi-leaf collimator and a first preset overlap range; control the leaves of the upper multi-leaf collimator and the leaves of the lower multi-leaf collimator to move based on the first target displacements and the third target displacements, respectively, to form the radiation field; determine second target displacements of the leaf heads of the upper multi-leaf collimator based on contour information of the radiation field and the contour information of the target radiation field; determine fourth target displacements of the leaf heads of the lower multi-leaf collimator based on the second target displacements and a second preset overlap range; and control the leaf heads of the upper multi-leaf collimator and the leaf heads of the lower multi-leaf collimator to move based on the second target displacements and the fourth target displacements, respectively, to adjust the radiation field.

In some embodiments, the upper multi-leaf collimator includes at least one first sensor and at least one second sensor. The at least one first sensor is configured to detect first actual displacements of the leaves of the upper multi-leaf collimator, and the at least one second sensor is configured to detect second actual displacements of the leaf heads. The lower multi-leaf collimator includes at least one third sensor and at least one fourth sensor. The at least one third sensor is configured to detect third actual displacements of the leaves of the lower multi-leaf collimator, and the at least one fourth sensor is configured to detect fourth actual displacements of the leaf heads of the lower multi-leaf collimator. The processing device is further configured to: adjust the first preset overlap range based on the first actual displacements and the third actual displacements; and adjust the second preset overlap range based on the second actual displacements and the fourth actual displacements.

According to an aspect of the present disclosure, a method for controlling the multi-leaf collimator is provided. The method may be implemented on a computing device having at least one processor and at least one storage device. The method comprises for each leaf, determining a first target displacement of the leaf based on contour information of a target radiation field corresponding to a target object; and controlling the first driving mechanism to drive the leaf to move based on the first target displacement to form the radiation field; for each leaf head, determining a second target displacement of the leaf head based on contour information of the radiation field and the contour information of the target radiation field; and controlling the second driving mechanism to drive the leaf head to move based on the second target displacement to adjust the radiation field.

In some embodiments, the method further comprises obtaining optical image data of the multi-leaf collimator that is collected after motions of the plurality of leaves; and determining the contour information of the radiation field based on the optical image data.

BRIEF DESCRIPTION OF THE DRAWINGS

The present disclosure is further illustrated in terms of exemplary embodiments. These exemplary embodiments are described in detail with reference to the drawings. These embodiments are not limiting, and in these embodiments, the same numbering denotes the same structure, wherein:

FIG. 1A is a diagram illustrating an exemplary structure of an MLC according to some embodiments of the present disclosure;

FIG. 1B is a top view illustrating a leaf portion of the MLC shown in FIG. 1A;

FIG. 2 is a diagram illustrating a portion of an exemplary MLC according to some embodiments of the present disclosure;

FIG. 3 is a diagram illustrating an exemplary leave according to some embodiments of the present disclosure;

FIG. 4 is a diagram illustrating an exemplary structure of an enlarged region A in FIG. 3 according to some embodiments of the present disclosure;

FIG. 5 is a diagram illustrating an exemplary structure of another MLC according to some embodiments of the present disclosure;

FIG. 6 is a diagram illustrating an exemplary structure of a second driving mechanism according to some embodiments of the present disclosure;

FIG. 7 is a diagram illustrating an exemplary structure of a second driving mechanism according to some embodiments of the present disclosure;

FIG. 8 is a diagram illustrating an exemplary structure of a double-layered MLC according to some embodiments of the present disclosure;

FIG. 9 is a diagram illustrating an exemplary structure of another MLC according to some embodiments of the present disclosure;

FIG. 10 is a flowchart illustrating a control method for an MLC according to some embodiments of the present disclosure;

FIG. 11 is a flowchart illustrating a control method for a second driving mechanism according to some embodiments of the present disclosure; and

FIG. 12 is a flowchart illustrating a control method for a double-layer MLC according to some embodiments of the present disclosure.

## DETAILED DESCRIPTION

In the following detailed description, numerous specific details are set forth by way of examples in order to provide a thorough understanding of the relevant disclosure. However, it should be apparent to those skilled in the art that the present disclosure may be practiced without such details. In other instances, well-known methods, procedures, systems, components, and/or circuitry have been described at a relatively high level, without detail, in order to avoid unnecessarily obscuring aspects of the present disclosure. Various modifications to the disclosed embodiments will be readily apparent to those skilled in the art, and the general principles defined herein may be applied to other embodiments and applications without departing from the spirit and scope of the present disclosure. Thus, the present disclosure is not limited to the embodiments shown, but to be accorded the widest scope consistent with the claims.

In the following detailed description, numerous specific details are set forth by way of examples in order to provide a thorough understanding of the relevant disclosure. However, it should be apparent to those skilled in the art that the present disclosure may be practiced without such details. In other instances, well-known methods, procedures, systems, components, and/or circuitry have been described at a relatively high level, without detail, in order to avoid unnecessarily obscuring aspects of the present disclosure. Various modifications to the disclosed embodiments will be readily apparent to those skilled in the art, and the general principles defined herein may be applied to other embodiments and applications without departing from the spirit and scope of the present disclosure. Thus, the present disclosure is not limited to the embodiments shown, but to be accorded the widest scope consistent with the claims.

The terminology used herein is for the purpose of describing particular example embodiments only and is not intended to be limiting. As used herein, the singular forms "a," "an," and "the" may be intended to include the plural forms as well, unless the context clearly indicates otherwise. It will be further understood that the terms "comprise," "comprises," and/or "comprising," "include," "includes," and/or "including," when used in this specification, specify the presence of stated features, integers, steps, operations, elements, and/or components, but do not preclude the presence or addition of one or more other features, integers, steps, operations, elements, components, and/or groups thereof.

It will be understood that the terms "system," "engine," "unit," "module," and/or "block" used herein are one method to distinguish different components, elements, parts, sections or assemblies of different levels in ascending order. However, the terms may be displaced by another expression if they achieve the same purpose.

It will be understood that when a unit, engine, module, or block is referred to as being "on," "connected to," or "coupled to," another unit, engine, module, or block, it may be directly on, connected or coupled to, or communicate with the other unit, engine, module, or block, or an intervening unit, engine, module, or block may be present, unless the context clearly indicates otherwise. As used herein, the term "and/or" includes any and all combinations of one or more of the associated listed items.

It will be understood that, although the terms "first," "second," "third," "fourth," etc., may be used herein to describe various elements, these elements should not be limited by these terms. These terms are only used to distinguish one element from another. For example, a first element could be termed a second element, and, similarly, a second element could be termed a first element, without departing from the scope of example embodiments of the present invention.

Spatial and functional relationships between elements (for example, between crystal elements) are described using various terms, including "connected," "engaged," "interfaced," and "coupled." Unless explicitly described as being "direct," when a relationship between first and second elements is described in the present disclosure, that relationship includes a direct relationship where no other intervening elements are present between the first and second elements, and also an indirect relationship where one or more intervening elements are present (either spatially or functionally) between the first and second elements. In contrast, when an element is referred to as being "directly" connected, engaged, interfaced, or coupled to another element, there are no intervening elements present. Other words used to describe the relationship between elements should be interpreted in a like fashion (e.g., "between," versus "directly between," "adjacent," versus "directly adjacent," etc.). As used herein, the term "and/or" includes any and all combinations of one or more of the associated listed items.

An MLC is configured to adjust a shape and a size of a radiation field by shielding and shaping radiation rays emitted from a radiation source. The MCL usually includes pairs of leaves, which are set up in two opposite groups. During a radiation treatment, each leaf moves independently along a length direction of the leaf (which is also referred to as an extension direction), thus creating a closed radiation field, so that healthy tissues around a tumor are not exposed to the radiation rays. However, due to the constraints of the MLC's transmission mechanism, the thickness of its leaves cannot be made too thin, which often results in relatively low resolution for the MLC and low targeting accuracy with respect to the tumor. Some embodiments of the present disclosure provide an MLC and a double-layer MLC, which have high resolutions and can accurately form the radiation field for the radiation treatment, thereby improving a therapeutic effect while reducing harm to the patient during the treatment.

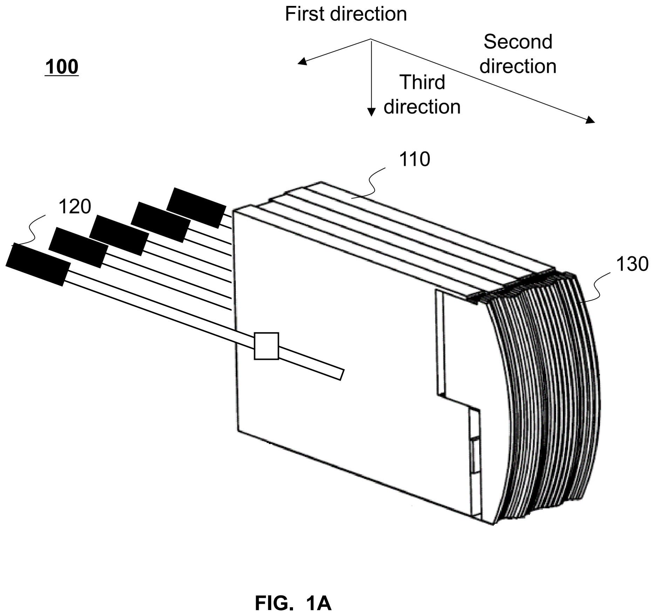

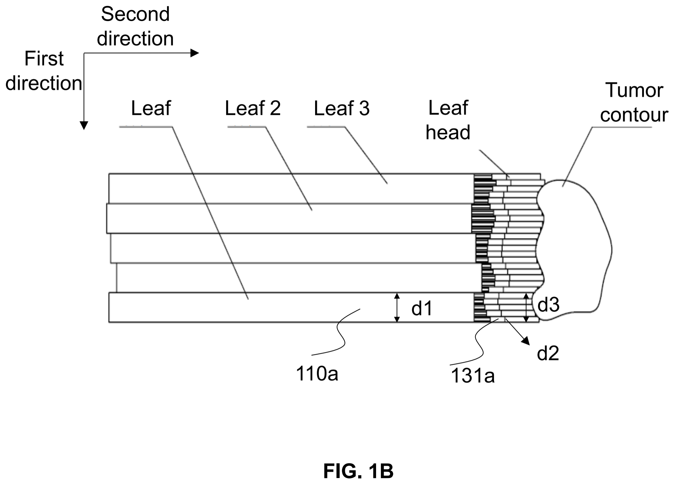

FIG. 1A is a diagram illustrating an exemplary structure of an MLC 100 according to some embodiments of the present disclosure. FIG. 1B is a top view illustrating a leaf portion of the MLC 100. For illustration purposes, only the structure of the MLC 100 on one side of a target object to be treated is shown. It should be understood that a similar structure is also provided on the opposite side of the target object. The coordinated arrangement of the structures on both sides of the target object can form a radiation field.

As shown in FIGs. 1A and 1B, the MLC 100 may include a plurality of leaves 110, first driving mechanisms 120, and at least one leaf head mechanism 130.

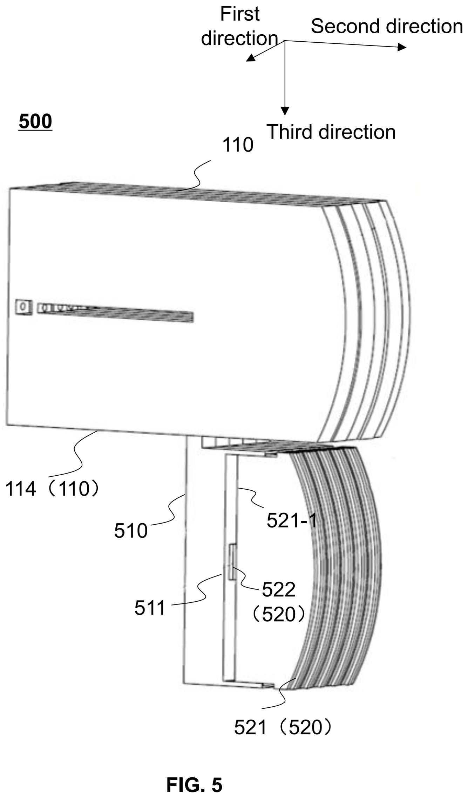

The plurality of leaves 110 are leaf-like mechanisms. In some embodiments, the at least one leaf head mechanism 130 is mounted on at least one corresponding leaf of the leaves and configured to adjust the radiation field. Each leaf head mechanism 130 is mounted on one leaf 110. In some embodiments, each leaf has at least one corresponding leaf head mechanism. For example, as the MLC 100 shown in FIG. 1A, each leaf 110 has its corresponding leaf head mechanism 130. As another example, a leaf (e.g., each leaf) may have a corresponding leaf head mechanism 130 as shown in FIG. 1 and a corresponding leaf head mechanism 520 as shown in FIG. 5. In some embodiments, only some leaves 110 have their corresponding leaf head mechanisms.

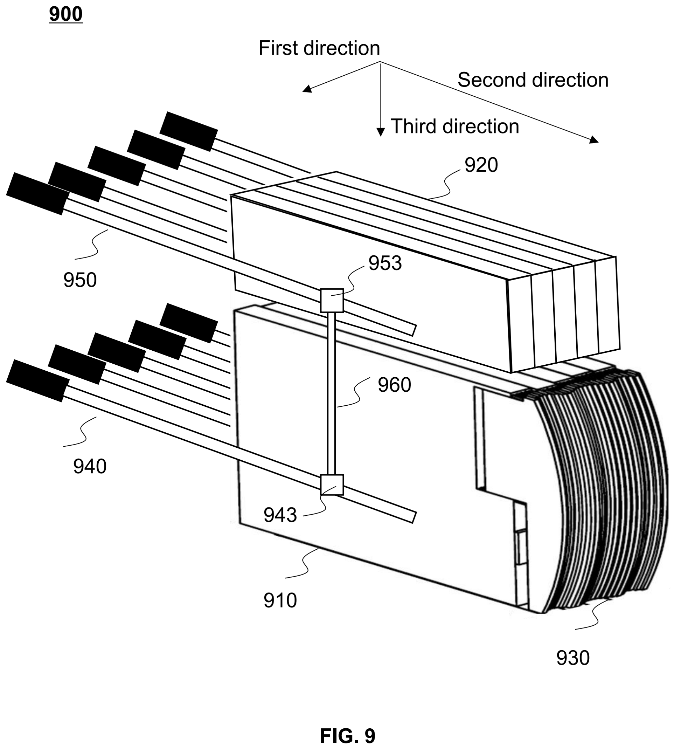

In some embodiments, the leaf 110 includes radiation shielding material (e.g., tungsten alloy) for shielding radiation rays emitted from a radiation source to form the radiation field. In some embodiments, the leaf 110 does not include the radiation shielding material, and serves only as an auxiliary device for mounting a leaf head mechanism and carrying the leaf head mechanism to move. In such cases, the MLC 100 further includes second leaves including the radiation shielding material, and the radiation field is formed by the second leaves. More about the second leaves may be found in the related descriptions of FIG. 9.

The leaves 110 are disposed side-by-side along a first direction, and each of the leaves 110 extends in a second direction. The first direction refers to a direction in which the leaves are arranged side-by-side, the second direction refers to a direction in which the leaves extend, and the second direction is perpendicular to the first direction. In some embodiments, the first direction is parallel to a thickness direction of the leaves 110, and the second direction is parallel to the length direction and the movement direction of the leaves 110. Each leaf 110 moves in the second direction to move closer to and away from a radiation field center. In some embodiments, the radiation field center is determined at a treatment planning stage based on information about the target object to be treated (such as a tumor region). For example, the radiation field center is coincident with an isocenter of the target object to be treated.

In some embodiments, in the second direction, a leaf 110 includes a first end close to the radiation field center and a second end away from the radiation field center; and in a third direction, the leaf 110 includes a third end close to the radiation source and a fourth end away from the radiation source. The third direction is perpendicular to a plane formed by the first direction and the second direction, and is parallel to a width direction of the leaf 110. In some embodiments, the third direction is parallel to a line connecting the radiation source and the radiation field center.

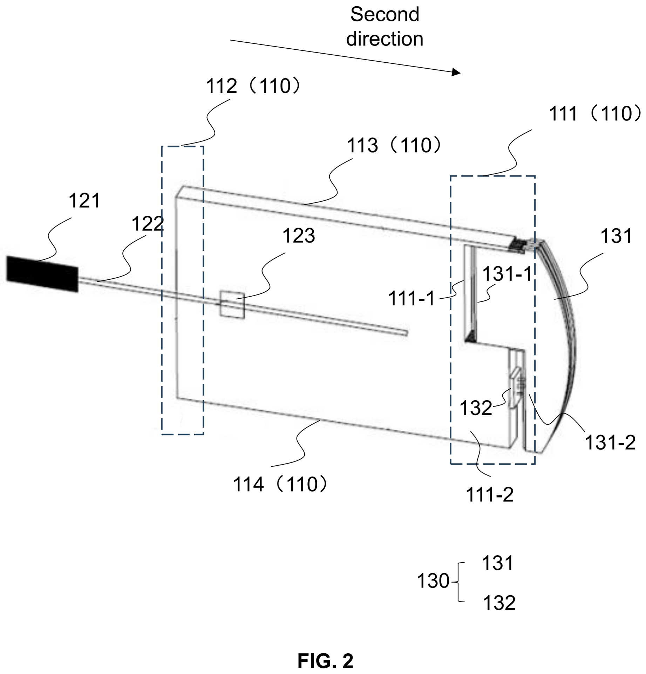

For illustration purposes, FIG. 2 provides a diagram illustrating a portion of the MLC 100 according to some embodiments of the present disclosure. As shown in FIG. 2, the leaf 110 includes a first end 111 close to the radiation field center, a second end 112 away from the radiation field center, a third end 113 close to the radiation source, and a fourth end 114 away from the radiation source.

The first driving mechanisms 120 are connected to the leaves 110 and configured to drive the leaves 110 to move along the second direction to close to or away from the radiation field center of the radiation field. In some embodiments, each of the first driving mechanisms 120 is connected to one leaf 110 and configured to drive the leaf 110 to which it is connected. For example, a first driving mechanism 120 is mounted to a leaf 110 at the second end 112 to drive the leaf 110 in the second direction to move close to or away from the radiation field center. Specifically, a positive direction of the second direction shown in the figures is a direction close to the radiation field center. The first driving mechanism 120 is connected to the leaf 110 in various ways, such as welding, bolted connection, riveting, etc.

In some embodiments, a first driving mechanism 120 includes a driving assembly (e.g., a motor), a transmission assembly (e.g., a driving screw), and a connection assembly (e.g., a nut). Merely by way of example, as shown in FIG. 2, the first driving mechanism 120 includes a driving assembly 121, a transmission assembly 122, and a connection assembly 123. The connection assembly 123 is connected to the leaf 110 and the transmission assembly 122. The driving assembly 121 drives the transmission assembly 122 and the connection assembly 123 to move, and the motion of the transmission assembly 122 and the connection assembly 123 drives the leaf 110 to move.

A leaf head mechanism 130 is a member for adjusting the radiation field. Each leaf head mechanism 130 is mounted on one leaf 110. In some embodiments, the leaf head mechanism 130 is mounted on the first end 111 of its corresponding leaf 110 as shown in FIG. 2. More descriptions of the leaf head mechanism 130 mounted on the first end 111 may be found in FIG. 2-FIG. 4 and their related descriptions. In some embodiments, the leaf head mechanism 130 is mounted on an end of the leaf 110 away from the radiation source, e.g., the fourth end 114. More descriptions of the leaf head mechanism 130 mounted on the fourth end 114 may be found in FIG. 5 and the related descriptions.

In some embodiments, the leaf 110 has two corresponding leaf head mechanisms 130, one leaf head mechanism 130 is mounted on the first end 111 of the leaf 110, and the other leaf head mechanism 130 is mounted on the fourth end 114 of the leaf 110.

In some embodiments, as shown in FIG. 2, the leaf head mechanism 130 mounted on the leaf 110 includes one or more leaf heads 131 and a second driving mechanism 132.

The leaf head(s) 131 include radiation shielding material and are configured to **precisely** adjust a size and a shape of the radiation field. With a thinner structural design and independent movement control of the leaf head(s), the radiation field can be adjusted more precisely. In some embodiments, the leaf head mechanism 130 includes one leaf head 131. To avoid radiation leakage, the thickness of the leaf head 131 is equal to or substantially equal to the thickness of the leaf 110. To save space, the width (i.e., the length along the third direction) of the leaf head 131 may be smaller than the width of the leaf 110.

In some embodiments, the leaf head mechanism 130 includes multiple leaf heads 131. The leaf heads 131 are arranged side-by-side along the first direction, with no gap between adjacent leaf heads. In some embodiments, each of the leaves 110 corresponds to at least two leaf heads 131. In some embodiments, the thickness of the each leaf head 131 is less than the thickness of the corresponding leaf 110, and a total thickness of the leaf heads 131 corresponding to the leaf 110 is equal to or substantially equal to the thickness of that leaf 110. For example, as shown in FIG. 1B, a thickness d2 of a leaf head 131a is less than a thickness d1 of a leaf 110a, and a total thickness d3 of the leaf heads corresponding to the leaf 110a is substantially equal to the thickness d1 of the leaf 110a.

In some embodiments, the thickness of each leaf head is less than 2.5mm. For example, the thickness of each leaf head is 1mm.

The second driving mechanism 132 is configured for driving a movement of the leaf heads 131 relative to the leaf 110. The second driving mechanism 132 is connected to the leaf heads 131 and is configured to drive the leaf heads 131 to move relative to the leaf 110, e.g., to move along the second direction. The second driving mechanism 132 is connected to the leaf heads 131 in various ways, such as welding, bolted connection, riveting, etc. In some embodiments, the second driving mechanism 132 is small in size and has a high degree of accuracy, enabling a millimeter or sub-millimeter level movement control of the leaf heads 131. Thus, the second driving mechanism 132 is also referred to as a micro-driving mechanism. In some embodiments, the second driving mechanism 132 is configured to individually drive each leaf head to move, for example, through control sub-films of the second driving mechanism 132 described below.

In application, the first driving mechanisms 120 separately drive the leaves 110 to move by the same distance or different distances along the second direction to initially form the radiation field. The motion of the leaves 110 drives their corresponding leaf head mechanisms 130 to move along the second direction. For each leaf head mechanism 130, the second driving mechanism 132 then drives the leaf heads 131 in the leaf head mechanism 130 to move for the same distance or different distances relative to the leaf 110 corresponding to the leaf head mechanism 130 in the second direction to adjust the radiation field, thus forming a final radiation field.

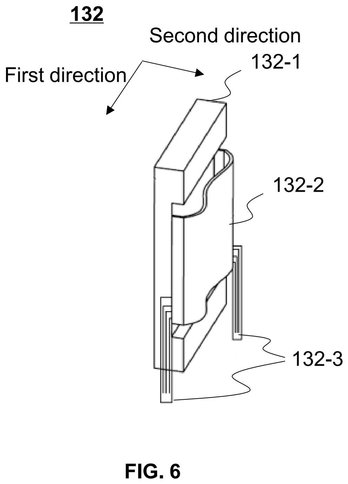

In some embodiments, the thickness of a leaf head 131 is less than its corresponding leaf 110, and the movement of the leaf head 131 can be adjusted more finely when the second driving mechanism drives the leaf head 131, so that the final radiation field formed by the leaf head 131 more closely conform to a shape of the target object to be treated. In some embodiments, the thickness of a leaf head 131 is the same as the thickness of the leaf 110, and the second driving mechanism 130 drives and adjusts the leaf head 131 so that the leaf head 131 is closer to the target object, resulting in a better radiation field conformity. As a result, by setting the leaf heads 131, an MLC with a higher resolution may be obtained, which can target the target object more precisely and reduce damage to surrounding healthy tissues in the radiation treatment. In some embodiments, the leaf heads are configured to move collectively with (move along with) the corresponding leaf driven by the first driving mechanism, and/or the leaf heads are configured to move independently driven by the second driving mechanism e.g., while the corresponding leaf is stationary, and the second driving mechanism is different from the first driving mechanism. More about the second driving mechanism may be found in FIGs. 6-7 and their related descriptions.

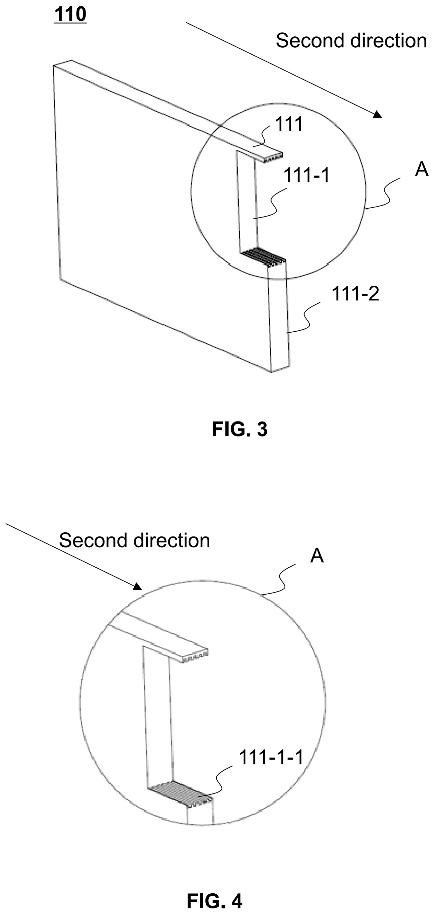

FIG. 3 is a diagram illustrating an exemplary leave 110 according to some embodiments of the present disclosure. As shown in FIG. 2 and FIG. 3, the first end 111 of the leaf 110 includes a first recess member 111-1 and an extension member 111-2. The extension member 111-2 is closer to the radiation field center relative to the first recess member 111-1.

The first recess member 111-1 is a structure for matching and mounting the leaf heads 131. In some embodiments, each leaf head 131 of the leaf head mechanism 130 includes a first protrusion member 131-1 that matches the first recess member 111-1. As shown in FIG. 2, the first recess member 111-1 of the leaf 110 matches the first protrusion members 131-1 of the leaf heads 131 to enable the leaf heads 131 to be mounted on the leaf 110.

In some embodiments, first motion guide grooves 111-1-1 extending along the second direction are provided within the first recess member 111-1. Merely by way of example, FIG. 4 is a diagram illustrating an exemplary structure of an enlarged region A in FIG. 3 according to some embodiments of the present disclosure. As shown in FIG. 4, the first motion guide grooves 111-1-1 extend in the second direction. A count of the first motion guide grooves 111-1-1 is the same as a count of the leaf heads 131 corresponding to the leaf 110. The first motion guide grooves 111-1-1 are configured to mount the first protrusion members 131-1 of the leaf heads 131 and to limit the motion direction of the leaf heads 131. Merely by way of example, as shown in FIG. 2, the first protrusion member 131-1 of each leaf head 131 is matched with the corresponding first motion guide groove 111-1-1, and limited by the first motion guide groove 111-1-1, the first protrusion member 131-1 can only move close to or away from the radiation field center along the second direction.

It is noted that a connection between the first recess member 111-1 of the leaf 110 and the first protrusion members 131-1 of the leaf heads 131 in FIG. 2 is merely illustrative, and the first recess member 111-1 can be matched and connected with the first protrusion members 131-1 in other ways, for example, via a pulley-groove connection.

The extension member 111-2 is configured to mount the second driving mechanism 132. As shown in FIG. 2, the second driving mechanism 132 is mounted on the extension member 111-2. The second driving mechanism 132 and the extension member 111-2 can be connected in various ways, such as welding, bolted connection, riveting, etc.

It should be noted that the mounting position of the second driving mechanism 132 in FIG. 2 is only for illustration purposes. An actual mounting position of the second driving mechanism 132 may be set according to actual needs. For example, the second driving mechanism 132 is mounted on a recess bottom surface of the first recess member 111-1.

In some embodiments, as shown in FIG. 2, each leaf head 131 further includes an abutting member 131-2 that is closer to the radiation field center relative to the first protrusion member 131-1. The second driving mechanism 132 connects to the abutting members 131-2 of the leaf heads 131 to drive the leaf heads 131 to move in the second direction relative to the leaf 110. The second driving mechanism 132 and the abutting members 131-2 are connected in various ways, such as welding, bolted connection, riveting, etc.

It should be noted that the shapes of the leaf 110 and the leaf heads 131 in FIG. 2 are only for illustration purposes. Actual shapes of the leaf 110 and the leaf heads 131 may be set according to actual needs. For example, the leaf 110 does not include the extension member 111-2, the leaf heads 131 do not include the abutting members 131-2, and the second driving mechanism 132 is mounted on the recess bottom surface of the first recess member 111-1 of the leaf 110, and is connected to the first protrusion members 131-1 of the leaf heads 131. For another example, as shown in FIG. 2, an end of each leaf head 131 near the radiation field center in the second direction protrudes relative to the first end 111 of the leaf 110. In some other embodiments, the end of each leaf head 131 near the radiation field center in the second direction flushes with the first end 111 of the leaf 110 (similar to the leaf heads 520 and the leaf 110 in FIG. 5). In such cases, which means that before the second driving mechanism 132 drives the leaf heads 131 to move relative to the leaf 110, a distance from the leaf heads 131 to the radiation field center and a distance from the leaf 110 to the radiation field center are the same.

In some embodiments, a count of the leaf heads 131 and a thickness of each leaf head 131 are set according to an actual situation, as long as the total thickness of the leaf heads 131 and a thickness of the corresponding leaf 110 are equal or approximately equal. Correspondingly, a count and widths of the first motion guide grooves 111-1-1 disposed within the first recess member 111-1 need to be set to match the count and the thicknesses of the leaf heads 131. It should be understood that the smaller the thicknesses of the leaf heads 131, the higher a resolution and a beam shaping accuracy, but a device cost and a production difficulty increase accordingly. Therefore, it is necessary to consider radiotherapy accuracy requirements and the device cost to select appropriate count and thicknesses.

In some embodiments, multiple MLCs 100 are produced. First recess members 111-1 and leaf head mechanisms 130 of different MLCs 100 correspond to different counts and thicknesses of leaf heads. In radiotherapy, a suitable MLC may be selected for use according to the radiotherapy accuracy requirements.

FIG. 5 is a diagram illustrating an exemplary structure of another MLC 500 according to some embodiments of the present disclosure. As shown in FIG. 5, each leaf head mechanism 520 of the MLC 500 is mounted on a fourth end 114 of the corresponding leaf 110. In some embodiments, the leaf head mechanism 520 is connected to the fourth end 114 of the corresponding leaf 110 in various ways, such as welding, bolted connection, riveting, etc.

In some embodiments, a suspension member 510 is disposed on the fourth end 114 of the leaf 110. The suspension member 510 is configured to mount the leaf head mechanism 520. In some embodiments, the suspension member 510 is fixedly mounted to the leaf 110 or integrally molded with the leaf 110. In some embodiments, the suspension member 510 is removably mounted to the leaf 110. In some embodiments, there are a plurality of suspension members 510, and each of the suspension members 510 is connected to one leaf 110 for mounting the leaf head mechanism 520 corresponding to the leaf 110 on the leaf 110. For illustration purposes, the following descriptions are provided with reference to one suspension member 510 and its corresponding leaf head mechanism 520 as an example.

In some embodiments, the suspension member 510 includes a second recess member 511 as shown in FIG. 5. The second recess member 511 is used for matching and mounting with the leaf heads 521 of the leaf head mechanism 520. In some embodiments, each leaf head 521 of the leaf head mechanism 520 includes a second protrusion member 521-1 that matches the second recess member 511. As shown in FIG. 5, the second recess member 511 of the suspension member 510 may match the second protrusion members 521-1 of the leaf heads 521 to enable the leaf heads 521 to be mounted on the suspension member 510.

In some embodiments, second motion guide grooves (not shown in the figures) extending in the second direction are disposed within the second recess member 511. The second motion guide grooves extend in the second direction, and a count of the second motion guide grooves is the same as the count of the leaf heads 521 corresponding to the leaf 110. The second motion guide grooves are configured to mount the second protrusion members 521-1 of the leaf heads 521 and to limit the motion direction of the leaf heads 521. Merely by way of example, as shown in FIG. 5, the second protrusion member 521-1 of each leaf head 521 is matched with the corresponding second motion guide groove, and is only able to move closer to or away from the radiation field center in the second direction limited by the second motion guide groove.

It should be noted that the connection between the second recess member 511 of the suspension member 510 and the second protrusion members 521-1 of the leaf heads 521 in FIG. 5 is shown for illustration purposes only. The second recess member 511 may also be matched with and connected to the second protrusion members 521-1 in other ways, for example, via a pulley-groove connection.

In some embodiments, the second driving mechanism 522 is mounted on a recess bottom surface of the second recess member 511. The second driving mechanism 522 and the recess bottom surface may be connected in various ways, such as welding, bolted connection, riveting, etc.

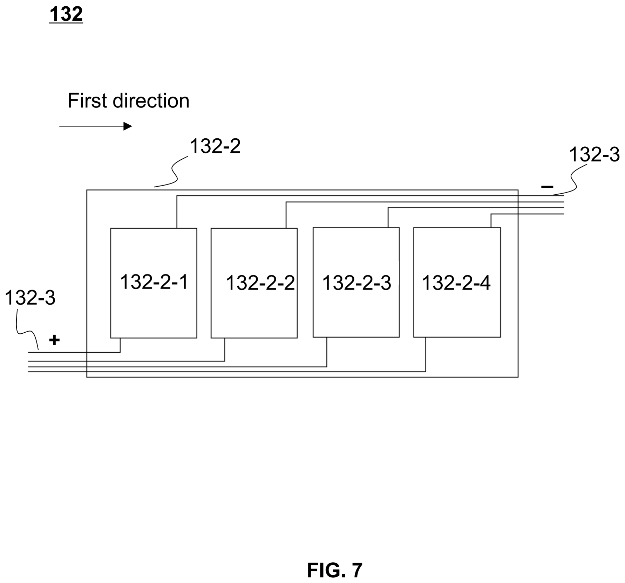

In some embodiments, the second driving mechanism 522 is connected to the second protrusion members 521-1 of the leaf heads 521 to drive the leaf heads 521 to move in the second direction relative to the leaf 110. The second driving mechanism 522 and the leaf heads 521 are connected in various ways, such as welding, bolted connection, riveting, etc. In some embodiments, the structure of the second driving mechanism 522 is similar to the structure of the second driving mechanism 132, which will be described in detail with reference to FIG. 6 and FIG. 7.

It should be noted that the shapes of the suspension member 510 and the leaf heads 521 in FIG. 5 are only for illustration purposes. Actual shapes of the suspension member 510 and the leaf heads 521 may be set according to actual needs.

In application, the MLC 500 can be controlled in a similar manner to how the MLC 100 is controlled as described above. The leaf 110 is driven by the first driving mechanism to carry the leaf head mechanism 520 to move toward the radiation field center to initially form the radiation field, and the leaf heads 521 is driven by the second driving mechanism 522 to further move relative to the leaf 110 in the second direction to form the final radiation field.

In some embodiments, the count of the leaf heads 521 and a thickness of each leaf head 521 are set according to actual situations, as long as a total thickness of the leaf heads 521 is equal to or approximately equal to a thickness of the corresponding leaf 110. Correspondingly, a count and widths of the second motion guide grooves disposed within the second recess member 511 of the suspension member 510 need to be set to match a count and thicknesses of the leaf heads 521. In some embodiments, a plurality of sets, each including a suspension member 510 and a corresponding leaf head mechanism 520, are prepared for the same leaf. Different sets correspond to different leaf head counts and leaf head thicknesses. In radiotherapy, a suitable set of the suspension member 510 and the leaf head mechanism 520 may be selected to be mounted on the leaf 110 according to the radiotherapy accuracy requirements.

FIG. 6 is a diagram illustrating an exemplary structure of a second driving mechanism 132 according to some embodiments of the present disclosure. As shown in FIG. 6, the second driving mechanism 132 includes a base 132-1, a control film 132-2, and a control power source 132-3.

The base 132-1 is configured to support and mount other components of the second driving mechanism 132. For example, the control film 132-2 and the control power supply 132-3 are mounted on the base 132-1 in various ways, such as welding, bolted connection, riveting, etc.

The control power supply 132-3 is configured to apply a voltage to the control film 132-2 to make the control film 132-2 produce a target deformation. The target deformation of the control film 132-2 drives the leaf heads 131 to move in the second direction. The target deformation is a deformation required by a radiation treatment on the control film, and the target deformation drives the leaf heads 131 to produce the required motion in the second direction to create a desired final radiation field. For example, the target deformation includes a bending direction, a bending degree, etc., of the control film in the radiation treatment.

The control film 132-2 is a structure configured for driving the motions of the leaf heads. The control film 132-2 is connected to the leaf heads 131.

In some embodiments, the control film 132-2 includes an artificial muscle material, for example, an ion-exchange polymer metal composite (IPMC) material. Due to the advantages of IPMC material-such as large deformation range, low noise during deformation, excellent flexibility, lightweight, low driving voltage, and fast/sensitive response, the control film fabricated by the IPMC material can quickly respond to the voltage applied by the control power source 132-3 and produce the target deformation. In some embodiments, the control film 132-2 includes a memory alloy material, for example, a nickel-titanium-based shape memory alloy, a copper-based shape memory alloy, etc.

In some embodiments, the bending direction of the control film 132-2 is changed by reversing the polarity (positive/negative) of the voltage input from the control power supply 132-3 to the control film 132-2. In some embodiments, the bending degree of the control film 132-2 is adjusted by varying the magnitude of the voltage applied by the control power supply 132-3.

In some embodiments, other controllable flexible materials, for example, responsive hydrogels, shape memory polymers, electroactive polymers, piezoelectric ceramics, etc., can also be employed to prepare the control film 132-2.

In some embodiments, the control film 132-2 includes control sub-films arranged side-by-side in the first direction. Each control sub-film is connected to one or more leaf heads. The control power supply 132-3 is configured to apply a voltage to each control sub-film individually to make the control sub-films move in combination to form the target deformation. Adjacent control sub-films may be interconnected (e.g., welded or bonded). When the control sub-films are controlled separately, different control sub-films may produce different motions. A combined motion of the control sub-films may produce the target deformation across the whole control film 132-2, which makes the leaf heads 131 move to adjust the radiation field.

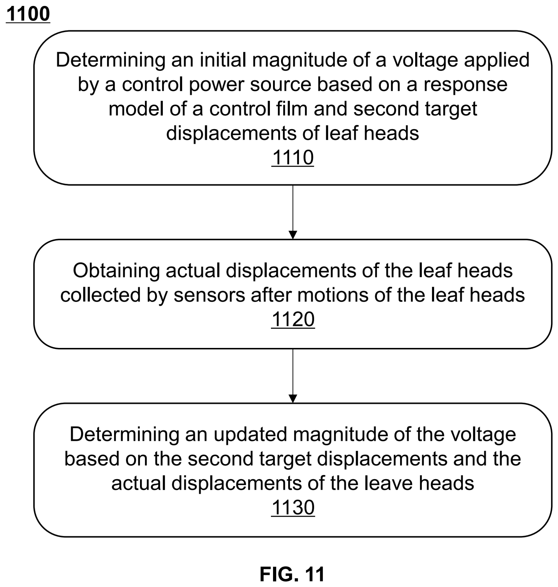

FIG. 7 is a diagram illustrating an exemplary structure of a second driving mechanism according to some embodiments of the present disclosure. As shown in FIG. 7, the control film 132-2 includes a first control sub-film 132-2-1, a second control sub-film 132-2-2, a third control sub-film 132-2-3, and a fourth control sub-film 132-2-4 arranged side-by-side in the first direction. The control power supply 132-3 is connected to and applies a voltage to the first control sub-film 132-2-1, the second control sub-film 132-2-2, the third control sub-film 132-2-3, and the fourth control sub-film 132-2-4, respectively, to make a combined deformation of these control sub-films form the target deformation. The voltages applied by the control power supply 132-3 to the different control sub-films may be the same or different. A detailed description of the control method of the second driving mechanism may be found in FIG. 11 and the related descriptions.

Some embodiments of the present disclosure provide a double-layer MLC. The double-layer MLC includes an upper MLC and a lower MLC arranged side-by-side in the third direction. At least one (e.g., each) of the upper MLC and the lower MLC is an MLC as described in any one of the foregoing embodiments.

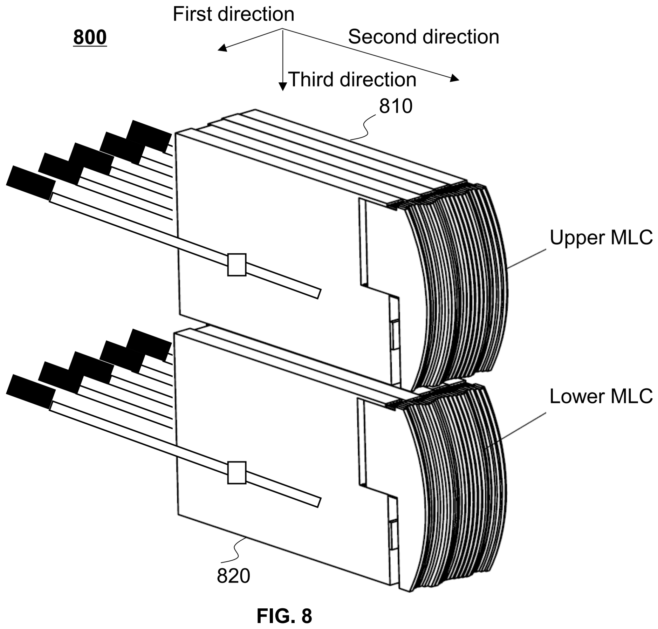

For illustration purposes only, FIG. 8 is a diagram illustrating an exemplary structure of a double-layered MLC 800 according to some embodiments of the present disclosure. As shown in FIG. 8, the double-layer MLC 800 includes an upper MLC 810 and a lower MLC 820 arranged side-by-side in the third direction. The upper MLC 810 is closer to a radiation source compared to the lower MLC 820. Both the upper MLC 810 and the lower MLC 820 are similar to the MLC 100 in FIG. 1. Leaves of the upper MLC 810 (also referred to as upper leaves) and leaves of the lower MLC 820 (also referred to as lower leaves) have a one-to-one correspondence. For an upper leaf and a lower leaf that correspond to each other, the leaf heads of the leaf head mechanisms thereof also have a one-to-one correspondence.

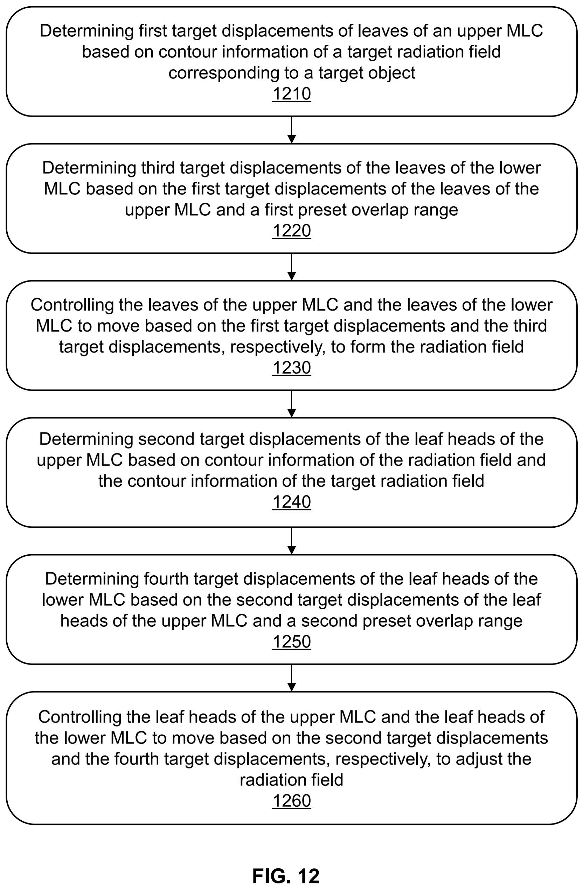

In application, the upper MLC 810 and the lower MLC 820 may form separate radiation fields. The upper MLC 810 is configured to initially filter radiation rays emitted by the radiation source, and the lower MLC 810 may further filter the radiation rays filtered by the upper MLC 810, so that the radiation rays emitted to the target object have a higher accuracy. A detailed description of the control method of the double-layer MLC 800 may be found in FIG. 12 and the related descriptions.

It should be noted that the double-layer MLC 800 shown in FIG. 8 is only illustrative, and one or more of the upper MLC and the lower MLC in the double-layer MLC may also be the MLC 500 shown in FIG. 5. By arranging the MLCs side by side in the third direction, a resolution of the MLCs is further improved, thereby improving an effectiveness of the radiation treatment and reducing side effects on a patient.

FIG. 9 is a diagram illustrating an exemplary structure of another MLC according to some embodiments of the present disclosure. As shown in FIG. 9, an MLC 900 includes leaves (also referred to as first leaves) 910, second leaves 920, leaf head mechanisms 930, first driving mechanisms 940, and third driving mechanisms 950. The first leaves 910 and the second leaves 920 are arranged side by side in a third direction. The second leaves 920 are closer to a radiation source compared to the first leaves 910 (i.e., the second leaves 920 are upper leaves of the first leaves 910).

The first leaves 910 leaves are provided with corresponding leaf head mechanisms 930. The first leaves 910 do not include radiation shielding material, and serve only as an auxiliary device for mounting the leaf head mechanisms 930 and carrying the leaf head mechanisms 930 to move. The first leaves 910 and the leaf head mechanisms 930 are similar to the leaves 110 and the leaf head mechanisms 130 in FIG. 1A-4, which are not repeated here.

The second leaves 920 include the radiation shielding material, and a radiation field is formed by the second leaves 920 after shielding the radiation rays emitted by the radiation source. In some embodiments, the second leaves 920 may or may not be provided with corresponding leaf head mechanisms.

The first driving mechanisms 940 are configured to drive the first leaves 910 to move closer to or further away from a radiation field center to form the radiation field. A structure of a first driving mechanism 940 is similar to a structure of a first driving mechanism 120, which is not repeated here. For example, the first driving mechanism 940 is connected to the first leaves 910 via a connection assembly 943 as shown in FIG. 9.

The third driving mechanisms 950 are configured to drive the second leaves 920 to move to form the radiation field. A structure of a third driving mechanism 950 is similar to the structure of a first driving mechanism 940, which is not repeated here. For example, the third driving mechanisms 950 are connected to the second leaves 920 via a connection assembly 953. Each third driving mechanism 950 is connected to one of the second leaves. The first leaves 910 and the second leaves 920 are in one-to-one correspondences, and accordingly, the first driving mechanisms 940 and the third driving mechanisms 950 are in one-to-one correspondences.

A first leaf and a second leaf corresponding to each other are synchronized to move, driven by the first driving mechanism and the third driving mechanism connected thereto. For example, either of the first driving mechanism 940 or the third driving mechanism 950 is a main driving mechanism and the other one is a secondary driving mechanism. It is assumed that the first driving mechanism 940 is the main driving mechanism and the third driving mechanism 950 is the secondary driving mechanism. When the first driving mechanism 940 drives the first leaf 910 connected thereto to move, the first driving mechanism 940 sends a synchronization signal to the corresponding second driving mechanism 950 to make the second driving mechanism 950 drive the corresponding second leaf 920 to move synchronously. Optionally, as shown in FIG. 9, the connection assembly 943 and the connection assembly 953 are connected by a transmission assembly 960, and the transmission assembly 960 is configured to improve the motion synchronization between the corresponding first leaf 910 and second leaf 920.

In some other embodiments, the third driving mechanism 950 is omitted. Each first driving mechanism 940 is further connected (e.g., bonded, welded, etc.) to one second leaf 920 and is configured to drive the synchronized motion of the first and second leaves to which it is connected.

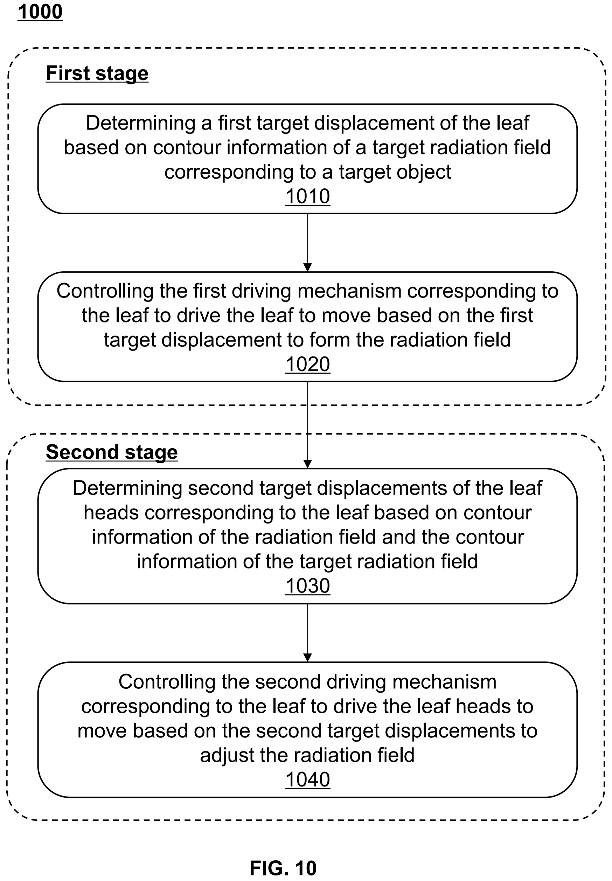

FIG. 10 is a flowchart illustrating a control method 1000 for an MLC according to some embodiments of the present disclosure. In some embodiments, the MLC (e.g., the MLC 100 or 500) further includes a processing device or is communicatively connected to the processing device, and the processing device is configured to perform the control method 1000. As shown in FIG. 10, the control method 1000 includes a first stage and a second stage. The first stage is for controlling the motion of leaves of the MLC to initially form a radiation field, which includes operations 1010 and 1020. The second stage is for controlling the motion of the leaf heads of the MLC to adjust the radiation field to form a final radiation field, which includes operations 1030 and 1040. The control method 1000 may be performed for each leaf. For illustration purposes, the following descriptions are provided with reference to the implementation of the control method 1000 for one leaf.

In operation 1010, the processing device determines a first target displacement of the leaf based on contour information of a target radiation field corresponding to a target object.

The target object is an object to be treated with radiation rays. For example, the target object includes diseased organs (e.g., tumors) of a patient. The target radiation field refers to an ideal radiation region with a specific shape formed within the patient's body after the radiation rays are shaped by the MLC. The target radiation field is determined according to treatment requirements of the target object, the primary goal being to precisely deliver radiation coverage to the target object while minimizing damage to surrounding healthy tissues.

The contour information of the target radiation field refers to information related to a boundary of the target radiation field. The contour information of the target radiation field includes data describing a shape and a position of the boundary of the target radiation field, for example, (x, y, z) coordinates of each boundary point in a three-dimensional (3D) coordinate system and parameters of boundary curves or surfaces.

In some embodiments, the processing device obtains the contour information of the target radiation field based on medical imaging data of the patient. Specifically, the processing device produces a 3D model of the target object and the surrounding tissues based on the medical imaging data of the patient via a 3D reconstruction technique. The medical imaging data of the patient refers to data obtained by a medical imaging device. For example, the medical imaging data includes tomographic computed tomography (CT) imaging data obtained by a CT imaging device, magnetic resonance (MRI) imaging data obtained by an MRI device, etc. Further, the processing device determines the contour information of the ideal target radiation field based on the 3D model via a treatment planning system (TPS) and simultaneously obtains a dose distribution within the target radiation field.

Then, the processing device determines a first target displacement of each leaf based on the contour information of the target radiation field, so that a contour of the radiation region within the patient after the motions of the leave (i.e., the radiation field actually formed by the MLC) is as closely as possible to a contour of the target radiation field. Each leaf's first target displacement indicates the distance and direction it needs to move relative to its current position. Specifically, the processing device employs an inverse kinematics algorithm to derive the target position of each leaf based on the contour information of the target radiation field. The first target displacement of each leaf is then determined according to its current position and target position. In some embodiments, each leaf is located at a preset initial position before the radiation treatment starts, and the leaves located on the same side of the target object are aligned. The first target displacement refers to a distance that each leaf moves toward the radiation field center of the target radiation field (i.e., in a positive direction of the second direction).

In operation 1020, the processing device controls the first driving mechanism corresponding to the leaf to drive the leaf to move based on the first target displacement to form the radiation field.

For example, the first driving mechanism 120 is controlled to drive each leaf to move to the corresponding target position based on the first target displacement, thereby forming the radiation field. The radiation field is a radiation field initially formed by the leaves of the MLC 100 in the first stage. Limited by the resolution of the leaves, the radiation field formed therein may have a certain deviation from the target radiation field, which needs to be fine-tuned by further utilizing the leaf head mechanism with a higher resolution in the second stage.

It should be understood that, for the MLC 100 illustrated in FIG. 1, since the leaf head mechanism 130 is mounted on the first end of the leaf 110 near the radiation field center, when the leaf 110 approaches the radiation field center, the leaf head mechanism 130 also approaches the radiation field center and is closer to the radiation field center relative to the leaf 110. In such cases, the radiation field is actually formed by the leaf 110 and the leaf head mechanism 130 together.

In operation 1030, the processing device determines second target displacements of the leaf heads corresponding to the leaf based on contour information of the radiation field and the contour information of the target radiation field.

The leaf heads corresponding to the leaf refer to the leaf heads of the leaf head mechanism mounted on the leaf. The contour information of the radiation field relates to a boundary of the radiation field formed by the leaves after they move based on their corresponding first target displacements. The contour information of the radiation field may include data describing a shape and a position of the boundary of the radiation field.

In some embodiments, the processing device obtains optical image data of the MLC and determines the contour information of the radiation field based on the optical image data. The optical image data of the MLC is collected by an optical imaging device (e.g., a radar laser camera, a point cloud camera, a depth camera, etc.) after the motion of the leaves. Specifically, the processing device segments the leaves from the optical image data and determines the contour information of the radiation field formed by the leaves based on the segmentation result.

The second target displacement of a leaf head indicates the distance and the direction that the leaf head needs to move relative to its current position to adjust the contour of the radiation field, such that the adjusted radiation field closely conforms to the contour of the target radiation field. Specifically, the processing device determines a contour deviation of the radiation field from the target radiation field based on the contour information of the radiation field and the contour information of the target radiation field, and determines a compensatory displacement (i.e., the second target displacement) for each leaf head to correct this the contour deviation.

In some embodiments of the present disclosure, the analysis accuracy of the radiation field is enhanced by integrating high-precision optical imaging devices, thereby improving the precision of radiation field adjustment.

In operation 1040, the processing device controls the second driving mechanism corresponding to the leaf to drive the leaf heads to move based on the second target displacements to adjust the radiation field.

The second driving mechanism corresponding to the leaf refers to the second driving mechanism of the leaf head mechanism mounted on the leaf. In some embodiments, the processing device determines a magnitude of a voltage to be applied to the control film in the second driving mechanism based on the second target displacements, and applies the voltage with such magnitude to the control film via the control power supply to drive the leaf heads to move, thereby adjusting the radiation field. In some embodiments, the control film includes multiple control sub-films. For each control sub-film, the processing device determines a magnitude of the voltage to be applied to the control sub-film based on the second target displacement(s) corresponding to the one or more leaf heads connected to the control sub-film, and applies the voltage with such magnitude to the control sub-film via the control power supply to drive the leaf head(s) to move. Detailed descriptions of determining the magnitude of the voltage to be applied to the control film or the control sub-film may be found in FIG. 11 and the related descriptions.

In some embodiments of the present disclosure, by performing two stages of displacement control to the leaf and the leaf heads, respectively, the accuracy of the final formed radiation field is improved, and thus radiation treatment accuracy is improved.

In some embodiments, the MLC 900 illustrated in FIG. 9 is controlled by a control method similar to the control method 1000, with a difference that the first target displacement of a second leaf is determined in operation 1010, and the second leaf and its corresponding to first leaf are driven to move synchronously based on the first target displacement in operation 1020.

FIG. 11 is a flowchart illustrating a control method 1100 for a second driving mechanism according to some embodiments of the present disclosure. In some embodiments, an MLC (e.g., any one of the MLCs 100, 500, 900) further includes a processing device or is communicatively connected to the processing device. The processing device is configured to perform the control method 1100. As shown in FIG. 11, the control method 1100 includes the following operations. In some embodiments, the control method 1100 is performed to achieve operation 1040 as described in connection with FIG. 10.

In operation 1110, the processing device determines an initial magnitude of a voltage applied by a control power source based on a response model of a control film and second target displacements of leaf heads.

The response model of the control film reflects a relationship between the voltage (V) and a mechanical deformation ($\Delta$x) of the control film. A voltage-deformation calibration experiment may be performed on the control film to establish the response model. The response model may be linear (e.g., $\Delta$x=k$\cdot$V) or nonlinear (e.g., $\Delta x = k \cdot V^2 + b$, where k and b are material feature parameters).

Specifically, the processing device substitutes the second target displacements of the leaf heads (e.g., an average of the second target displacements) as a mechanical deformation ($\Delta$x) into the response model to obtain a voltage (V) as the initial magnitude of the voltage applied by the control power source.

In operation 1120, the processing device obtains actual displacements of the leaf heads collected by sensors after motions of the leaf heads.

The MLC 100 may further include the sensors. Each sensor is mounted on one leaf head and configured to measure a displacement of the leaf head. The sensors may include, for example, capacitive sensors, fiber optic sensors, etc. When a leaf head moves, a sensitive component (e.g., a capacitor or an optical grid) of the sensor on the leaf head has physical feature changes (such as changes in the capacitance and light intensity) with the movement of the leaf head, and the sensor converts the changes into an actual displacement of the leaf head.

In operation 1130, the processing device determines an updated magnitude of the voltage based on the second target displacements and the actual displacements of the leaf heads.

In some embodiments, the processing device determines a displacement deviation based on the second target displacement and the actual displacement of each leaf head, and determines an updated magnitude of the voltage based on the displacement deviation of each leaf head.

Merely by way of example, the processing device determines the updated magnitude of the voltage based on the response model and the displacement deviation of each leaf head. For example, an average displacement deviation of the leaf heads is substituted into the response model as a mechanical deformation to obtain a corresponding voltage adjustment value, and then the initial magnitude of the voltage is updated based on the voltage adjustment value to determine the updated magnitude of the voltage.

For another example, the processing device determines the updated magnitude of the voltage based on a proportional-integral-derivative control (PID) algorithm and the displacement deviation of each leaf head. In some embodiments, the PID algorithm includes a proportional coefficient, an integral coefficient, and a differential coefficient. For example, the PID algorithm is represented by formular (1):

$$V = P + I + D = K_p e(t) + K_i \int_0^t e(\tau)d\tau + K_d \frac{de(t)}{dt} \ (1)$$

where $V$ denotes the updated magnitude of the voltage, $P, I,$ and D denote a proportional term, an integral term, and a differential term of the PID algorithm, respectively, $e(t)$ denotes the displacement deviation at a current moment $t$ (i.e., a difference between the second target displacement and the actual displacement, as described above), and $e(\tau)$ denotes a displacement deviation at a time $\tau$ (where $\tau$ denotes a moment between an initial moment 0 and the current moment $t$). In some embodiments, $e(t)$ may be the average displacement deviation of the leaf heads at the current moment $t$, $e(\tau)$ may be the average displacement deviation of the leaf heads at the time $\tau$.

The proportional term $P$ is used to generate a control signal according to the displacement deviation $e(t)$, where the greater the displacement deviation, the stronger the control signal. The integral term $I$ is used to accumulate historical deviations (i.e., a sum of the displacement deviations from the initial moment 0 to the current moment $t$) to eliminate steady-state deviations (e.g., long-term deviations) that cannot be corrected by the proportional term. The differential term $D$ is used to measure a change rate of the displacement deviation, which inhibits a system oscillation and improves a system stability. $K_p$ denotes the proportional coefficient that determines a strength of the response to the displacement deviation e(t) at the current moment. $K_i$ is the integration coefficient, which determines a correction strength on an accumulated historical deviation. $K_d$ is the differential coefficient, which determines an ability to inhibits the change rate of the displacement deviation.

In some embodiments, the proportional coefficient $K_p$, the integration coefficient $K_i$, and the differential coefficient $K_d$ are system default settings, or set by a user, or determined through data analysis. For example, at least one of the proportional coefficient, the integral coefficient, and the differential coefficient are determined based on the response model. Specifically, $K_p$, $K_i$, and $K_d$ may be set based on a response sensitivity (e.g., $\Delta x/V$), which is derived from the response model. For example, the system with high sensitivity (i.e., greater $\Delta x/V$) needs to a lower $K_p$ to avoid overshoot. The system with hysteresis (i.e., smaller $\Delta x/V$) or a nonlinear system needs to increase the integration term $K_i$ to eliminate a steady-state error. By adjusting $K_p$, $K_i$, and $K_d$, a response speed and a stability of the PID algorithm may be optimized.

In some embodiments of the present disclosure, based on the PID algorithm, the updated magnitude of the voltage is accurately determined to improve a control accuracy of the control film. In addition, by determining the coefficients of the PID algorithm based on the response model, the PID algorithm is made more in line with the features of the control film, and an accuracy of voltage adjustment is improved.

In some embodiments of the present disclosure, the sensors are introduced to monitor the actual displacements of the leaf heads, thereby forming a negative feedback mechanism to adjust the voltage applied to the control film, which makes the control of the control film more accurate and thus improves the accuracy of radiation field adjustment.

In some embodiments, the control film includes control sub-films disposed side-by-side along the first direction, and each control sub-film is connected to one or more leaf heads. The control power supply applies a voltage to each of the control sub-films individually to make them to drive the motion of the connected leaf head(s), respectively. In operation 1110, the processing device determines the initial magnitude of the voltage that the control power supply applies to each control sub-film. Specifically, for each control sub-film, the processing device takes a first average value of the second target displacements of the one or more leaf heads connected thereto as the mechanical deformation ($\Delta x$) to substitute into the response model to obtain a voltage (V) as the initial magnitude of the voltage applied to the control sub-film. In operation 1130, the processing device determines the updated magnitude of the voltage applied to each control sub-film. Specifically, for each control sub-film, the processing device may determine a second average value of the actual displacements of the one or more leaf heads connected thereto, and take a difference between the first average value and the second average value as a corresponding displacement deviation of the control sub-film. The processing device may further determine the updated magnitude of the voltage corresponding to the control sub-film based on the corresponding displacement deviation of the control sub-film. By setting the control sub-films to separately drive different leaf heads, the control accuracy of the leaf heads may be improved, thereby improving the accuracy of the radiation field adjustment.

FIG. 12 is a flowchart illustrating a control method 1200 for a double-layer MLC 800 according to some embodiments of the present disclosure. In some embodiments, the double-layered MLC 800 further includes a processing device or is communicatively connected to the processing device, the processing device is configured to perform the control method 1200. As shown in FIG. 12, the control method 1200 includes following operations.

In operation 1210, the processing device determines first target displacements of leaves of an upper MLC based on contour information of a target radiation field corresponding to a target object.

Operation 1210 may be performed in a similar manner as operation 1010, and the descriptions thereof are not repeated here.

In operation 1220, the processing device determines third target displacements of the leaves of the lower MLC based on the first target displacements of the leaves of the upper MLC and a first preset overlap range.

The first preset overlap range refers to an offset distance of the leaves of the lower MLC (hereinafter referred to as the lower leaves) relative to the leaves of the upper MLC (hereinafter referred to as the upper leaves), which provide dual shielding of the radiation rays at the edges of the radiation field. After the radiation rays passed through the upper leaves, potential leakage may occur at the edges of the radiation field near the upper leaves, and the lower leaves may provide secondary shielding for such leaked radiation rays. In some embodiments, the first preset overlap range is set by a user, or according to a system default setting, or determined based on statistical data of leaf displacement errors. For example, the first preset overlap range is 5 mm.

As shown in FIG. 8, the upper leaves and the lower leaves are in a one-to-one correspondence. For each lower leaf, the processing device may superimpose the first preset overlap range on the first target displacement of the upper leaf corresponding thereto to obtain the third target displacement of the lower leaf. It should be understood that a lower leaf has the same displacement direction as its corresponding upper leaf, and the lower leaf is closer to the radiation field center, thereby providing the secondary shielding for the radiation rays leaked from the upper leaf.

In some embodiments, the upper MLC includes one or more first sensors and the lower MLC includes one or more third sensors. When the upper leaves move, the first sensor(s) are configured to detect first actual displacements of the upper leaves. When the lower leaves move, the third sensor(s) are configured to detect third actual displacements of the lower leaves. The first sensor(s) and the third sensor(s) may be any type of displacement sensors (e.g., an optical sensor, a capacitive displacement sensor).

In some embodiments, the processing device adjusts the first preset overlap range based on the first actual displacements and the third actual displacements. Specifically, the processing device may determine a first actual overlap range based on the first actual displacements and the third actual displacements. The first actual overlap range refers to an actual offset distance of the upper leaves relative to the lower leaves after motion of the upper and lower leaves. For example, an actual offset distance between each upper leaf and its corresponding lower leaf is determined based on the first actual displacement and the third actual displacement of these two leaves, and an average of the actual offset distances corresponding to the upper leaves is determined as the first actual overlap range.

If the first actual overlap range is smaller than the first preset overlap range but larger than a first threshold, the positions of the lower leaves may be adjusted so that the adjusted first actual overlap range is similar to the first preset overlap range. If the first actual overlap range is less than the first threshold (e.g., a minimum tolerable leaf overlap range), the first preset overlap range may be increased. Due to mechanical errors (such as a guide rail clearance, a thermal expansion, a control delay, etc.), the first actual overlap range may be smaller than the first preset overlap range. If the first actual overlap range is too small, it indicates that the originally set first preset overlap range is insufficient and needs to be increased. In this way, even if there is the mechanical errors, the actual first actual overlap range can still satisfy requirements and maintain a better radiation shielding effect.

In operation 1230, the processing device controls the leaves of the upper MLC and the leaves of the lower MLC to move based on the first target displacements and the third target displacements, respectively, to form the radiation field.

The upper leaves and the lower leaves are controlled in a manner similar to the manner of controlling the leaves as described in operation 1020, which is not repeated here.

In operation 1240, the processing device determines second target displacements of the leaf heads of the upper MLC based on contour information of the radiation field and the contour information of the target radiation field.

Operation 1240 may be performed in a similar manner as operation 1030, and the descriptions thereof are not repeated here.

In operation 1250, the processing device determines fourth target displacements of the leaf heads of the lower MLC based on the second target displacements of the leaf heads of the upper MLC and a second preset overlap range.

The fourth target displacement indicates a distance and a direction that each leaf head of the lower MLC needs to move relative to its current position, ensuring precise conformity between the shape of the radiation rays and the contour of the target radiation field.

The second preset overlap range refers to an offset distance of the leaf heads of the lower MLC (hereinafter referred to as the lower leaf heads) relative to the leaf heads of the upper MLC (hereinafter referred to as the upper leaf heads) for double-shielding the radiation rays at the edge of the radiation field. In some embodiments, the second preset overlap range is set by user, or according to a system default setting, or determined based on the statistical data of the leaf head displacement errors. For example, the second preset overlap range is 0.5 mm.

In some embodiments, the upper leaf heads and the lower leaf heads are in one-to-one correspondences. For each lower leaf head, the processing device superimposes the second preset overlap range on the corresponding second target displacement of the upper leaf head to obtain the fourth target displacement of the lower leaf head. It should be understood that a lower leaf heads has the same displacement direction as its corresponding upper leaf head, and the lower leaf head is closer to the radiation field center, thereby providing the secondary shielding for the radiation rays leaked from the upper leaf head.

In some embodiments, the upper MLC includes one or more second sensors and the lower MLC includes one or more fourth sensors. After the upper leaf heads move, the second sensor(s) are configured to detect second actual displacements of the upper leaf heads. After the lower leaf heads move, the fourth sensor(s) are configured to detect fourth actual displacements of the lower leaf heads. The second sensor(s) and the fourth sensor(s) may be any type of displacement sensors (e.g., the optical sensor and the capacitive displacement sensor). In some embodiments, the first sensor(s) and the third sensor(s) have lower resolutions compared to the second sensor(s) and the fourth sensor(s), given that a motion accuracy of the leaves is less than that of the leaf heads. For example, the first sensor(s) and the third sensor(s) are optical sensors with a resolution of 0.1 um, and the second sensor(s) and fourth sensor(s) are capacitive displacement sensors with a resolution of 0.01 um.

In some embodiments, the processing device adjusts the second preset overlap range based on the second actual displacements and the fourth actual displacements. Specifically, the processing device may determine a second actual overlap range based on the second actual displacement and the fourth actual displacement. The second actual overlap range is an actual offset distance of the upper leaf heads relative to the lower leaf heads after motion of the upper leaf heads and the lower leaf heads. If the second actual overlap range is smaller than the second preset overlap range but greater than a second threshold, positions of the lower leaf heads may be adjusted so that the adjusted second actual overlap range is similar to the second preset overlap range. If the second actual overlap range is less than the second threshold (e.g., a minimum tolerable leaf head overlap range), the second preset overlap range may be increased. A principle of increasing the second preset overlap range is similar to a principle of increasing the first preset overlap range described in operation 1220.

In operation 1260, the processing device controls the leaf heads of the upper MLC and the leaf heads of the lower MLC to move based on the second target displacements and the fourth target displacements, respectively, to adjust the radiation field.

The upper leaf heads and the lower leaf heads are controlled in a manner similar to the manner of controlling the leaf heads as described in operation 1040, which is not described here.

Some embodiments of the present disclosure may enable a synergistic control of multi-layer leaves and multi-layer leaf heads. Additionally, by introducing a feedback mechanism based on the actual displacements, the control accuracy of the multi-layer MLC and the treatment accuracy are further improved.

Also, the present disclosure uses specific words to describe embodiments thereof. "An embodiment", "one embodiment", and/or "some embodiments" means a feature, structure, or characteristic associated with at least one embodiment of the present disclosure. Accordingly, it should be emphasized and noted that "an embodiment" or "one embodiment" or "an alternative embodiment" in different places in the present disclosure do not necessarily refer to the same embodiment. In addition, certain features, structures, or characteristics of one or more embodiments of the present disclosure may be suitably combined.

Additionally, unless expressly stated in the claims, the order of the processing elements and sequences, the use of numerical letters, or the use of other names as described in the present disclosure are not intended to qualify the order of the processes and methods of the present disclosure. While some embodiments of the present disclosure that are currently considered useful are discussed in the foregoing disclosure by way of various examples, it is to be understood that such details serve only illustration purposes, and that the additional claims are not limited to the disclosed embodiments. Rather, the claims are intended to cover all amendments and equivalent combinations that are consistent with the substance and scope of the embodiments of the present disclosure. For example, while the above-described system components may be implemented by hardware devices, they may also be implemented by software-only solutions, such as installing the described system on existing servers or motion devices.

Similarly, it should be noted that to simplify the presentation of the disclosure of the present disclosure, and thereby aid in the understanding of one or more embodiments of the present disclosure, the foregoing descriptions of embodiments of the present disclosure sometimes combine a variety of features into a single embodiment, accompanying drawings, or descriptions thereof. However, this manner of disclosure does not imply that the objects of the present disclosure require more features than those mentioned in the claims. Rather, claimed subject matter may lie in less than all features of a single foregoing disclosed embodiment.

Some embodiments use numbers to describe the number of components, attributes, and it should be understood that such numbers used in the description of embodiments are modified in some examples by the modifiers "approximately", "nearly", or "substantially". Unless otherwise noted, the terms "approximately", "nearly", or "substantially" indicates that a $\pm 20\%$ variation in the stated number is allowed. Correspondingly, in some embodiments, the numerical parameters used in the present disclosure and the claims are approximations, and the approximations are subject to change depending on the desired characteristics of individual embodiments. In some embodiments, the numerical parameters should take into account the specified number of valid digits and use a general digit retention method. While the numerical domains and parameters configured to confirm the breadth of their ranges in some embodiments of the present disclosure are approximations, in specific embodiments such values are set to be as precise as possible within a feasible range.

For each of the patents, patent applications, patent application disclosures, and other materials cited in the present disclosure, such as articles, books, specification sheets, publications, documents, etc., the entire contents of which are hereby incorporated herein by reference. Application history documents that are inconsistent with or conflict with the contents of the present disclosure are excluded, as are documents (currently or hereafter appended to the present disclosure) that limit the broadest scope of the claims of the present disclosure. It should be noted that in the event of any inconsistency or conflict between the descriptions, definitions, and/or use of terms in the materials appurtenant to the present disclosure and those set forth herein, the descriptions, definitions and/or use of terms in the present disclosure shall prevail.

Finally, it should be understood that the embodiments described herein are only configured to illustrate the principles of the embodiments of the present disclosure. Other deformations may also fall within the scope of the present disclosure. As such, alternative configurations of embodiments of the present disclosure may be viewed as consistent with the teachings of the present disclosure as an example, not as a limitation. Correspondingly, the embodiments of the present disclosure are not limited to the embodiments expressly presented and described herein.

**Claims**

1. A multi-leaf collimator, comprising:

   a plurality of leaves;
   a first driving mechanism connected to the leaves and configured to drive the leaves to move to form a radiation field; and

at least one leaf head mechanism mounted on at least one corresponding leaf of the leaves and configured to adjust the radiation field.

2. The multi-leaf collimator of claim 1, wherein each leaf head mechanism includes:

   leaf heads, each of which has a thickness less than a thickness of the corresponding leaf; and
   a second driving mechanism configured to drive the leaf heads to move relative to the corresponding leaf.

3. The multi-leaf collimator of claim 2, wherein

   the corresponding leaf includes a first end close to the radiation field center, a second end away from the radiation field center, a third end close to a radiation source, and a fourth end away from the radiation source,
   the leaf head mechanism is mounted on the first end or the fourth end.

4. The multi-leaf collimator of claim 3, wherein the leaf head mechanism is mounted on the first end,

   the first end includes a first recess member, the first recess member including first motion guide grooves disposed inside,
   each leaf head includes a first protrusion member matching the first recess member, and
   the first motion guide grooves are configured to mount the first protrusion members of the leaf heads and limit a motion direction of the leaf heads.

5. The multi-leaf collimator of claim 4, wherein the first end further includes an extension member closer to the radiation field center relative to the first recess member,

   each leaf head further includes an abutting member closer to the radiation field center relative to its first protrusion member, and
   the second driving mechanism is mounted on the extension member and connected to the abutting members of the leaf heads to drive the leaf heads to move relative to the corresponding leaf.

6. The multi-leaf collimator of any one of claims 1-5, wherein the leaves include radiation shielding material, and the radiation field is formed by the leaves.

7. The multi-leaf collimator of any one of claims 2 to 6, wherein the second driving mechanism includes a base, a control film mounted on the base, and a control power supply,

   the control film is connected to the leaf heads,
   the control power supply is configured to apply a voltage to the control film to cause the control film to produce a target deformation, and the target deformation of the control film drives the leaf heads to move.

8. The multi-leaf collimator of claim 7, wherein the control film comprises a plurality of control sub-films arranged in parallel, and
   the control power supply is configured to individually apply the voltage to each control sub-film.

9. The multi-leaf collimator of claim 7 or 8 , wherein the multi-leaf collimator further includes a processing device and sensors, each of the sensors is mounted on one of the leaf heads, and the processing device is configured to:

   determine an initial magnitude of the voltage applied by the control power source based on a response model of the control film and second target displacements of the leaf heads, wherein the response model reflects a relationship between the voltage and a mechanical deformation of the control film;
   obtain actual displacements of the leaf heads collected by the sensors after motions of the leaf heads; and
   determine an updated magnitude of the voltage based on the second target displacements and the actual displacements of the leaf heads.

10. The multi-leaf collimator of any one of claims 2-9, wherein the leaf heads are configured to move collectively with the corresponding leaf driven by the first driving mechanism, or the leaf heads are configured to move independently driven by the second driving mechanism while the corresponding leaf is stationary, and the second driving mechanism is different from the first driving mechanism.

11. The multi-leaf collimator of any one of claims 2 to 10, wherein the second driving mechanism is configured to individually drive each leaf head to move.

12. A radiation therapy device, including the multi-leaf collimator of any one of claims 1 to 11.

13. A double-layer multi-leaf collimator, including an upper multi-leaf collimator and a lower multi-leaf collimator, wherein

> the upper multi-leaf collimator is closer to a radiation source than the lower multi-leaf collimator, and
> each of the upper multi-leaf collimator and the lower multi-leaf collimator is the multi-leaf collimator of claim 2.

14. A method for controlling the multi-leaf collimator of claim 10, implemented on a computing device having at least one processor and at least one storage device, the method comprising:

> for each leaf,

>> determining a first target displacement of the leaf based on contour information of a target radiation field corresponding to a target object; and
>> controlling the first driving mechanism to drive the leaf to move based on the first target displacement to form the radiation field;

> for each leaf head,

>> determining a second target displacement of the leaf head based on contour information of the radiation field and the contour information of the target radiation field; and
>> controlling the second driving mechanism to drive the leaf head to move based on the second target displacement to adjust the radiation field.

15. The method of claim 14, further comprising

> obtaining optical image data of the multi-leaf collimator that is collected after motions of the plurality of leaves; and
> determining the contour information of the radiation field based on the optical image data.

First direction

Second direction

Third direction

**100**

120

110

130

**FIG. 1A**

**FIG. 1B**

Second
direction

112（110）

111（110）

113（110）

121

122

123

111-1

131-1

131

114（110）

132

111-2

131-2

130 [ 131
       132 ]

**FIG. 2**

**110**

Second direction

111

111-1

A

111-2

**FIG. 3**

Second direction

A

111-1-1

**FIG. 4**

First
direction

Second direction

Third direction

**500**

110

114（110）

510

521-1

511

522
（520）

521（520）

**FIG. 5**

**132**

Second direction

First direction

132-1

132-2

132-3

**FIG. 6**

**132**

First direction

**FIG. 7**

**FIG. 8**

**FIG. 9**

1000

**First stage**

Determining a first target displacement of the leaf based on contour information of a target radiation field corresponding to a target object
1010

Controlling the first driving mechanism corresponding to the leaf to drive the leaf to move based on the first target displacement to form the radiation field
1020

**Second stage**

Determining second target displacements of the leaf heads corresponding to the leaf based on contour information of the radiation field and the contour information of the target radiation field
1030

Controlling the second driving mechanism corresponding to the leaf to drive the leaf heads to move based on the second target displacements to adjust the radiation field
1040

**FIG. 10**

**1100**

Determining an initial magnitude of a voltage applied by a control power source based on a response model of a control film and second target displacements of leaf heads
**1110**

Obtaining actual displacements of the leaf heads collected by sensors after motions of the leaf heads
**1120**

Determining an updated magnitude of the voltage based on the second target displacements and the actual displacements of the leave heads
**1130**

**FIG. 11**

Determining first target displacements of leaves of an upper MLC based on contour information of a target radiation field corresponding to a target object
1210

Determining third target displacements of the leaves of the lower MLC based on the first target displacements of the leaves of the upper MLC and a first preset overlap range
1220

Controlling the leaves of the upper MLC and the leaves of the lower MLC to move based on the first target displacements and the third target displacements, respectively, to form the radiation field
1230

Determining second target displacements of the leaf heads of the upper MLC based on contour information of the radiation field and the contour information of the target radiation field
1240

Determining fourth target displacements of the leaf heads of the lower MLC based on the second target displacements of the leaf heads of the upper MLC and a second preset overlap range
1250

Controlling the leaf heads of the upper MLC and the leaf heads of the lower MLC to move based on the second target displacements and the fourth target displacements, respectively, to adjust the radiation field
1260

**FIG. 12**

# EUROPEAN SEARCH REPORT

**Application Number**

EP 25 21 2653

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/265071 A1 (ROCHFORD RONAN [FI] ET AL) 26 August 2021 (2021-08-26) | 1-6, 10-15 | INV. A61N5/10 G21K1/04 |
| A | * paragraphs [0034], [0040] - [0044]; figures 1-4,7A-C * | 7-9 | |

-----

**TECHNICAL FIELDS SEARCHED (IPC)**

A61N
G21K

The present search report has been drawn up for all claims

| Place of search | Date of completion of the search | Examiner |
|---|---|---|
| Munich | 16 March 2026 | Kajzar, Anna |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

EP 4 736 941 A1

**ANNEX TO THE EUROPEAN SEARCH REPORT
ON EUROPEAN PATENT APPLICATION NO.**

EP 25 21 2653

This annex lists the patent family members relating to the patent documents cited in the above-mentioned European search report.
The members are as contained in the European Patent Office EDP file on
The European Patent Office is in no way liable for these particulars which are merely given for the purpose of information.

16-03-2026

| Patent document cited in search report | Publication date | Patent family member(s) | Publication date |
|---|---|---|---|
| US 2021265071 A1 | 26-08-2021 | US 2021265071 A1<br>WO 2021170722 A1 | 26-08-2021<br>02-09-2021 |

EPO FORM P0459

For more details about this annex : see Official Journal of the European Patent Office, No. 12/82